(19) Europäisches Patentamt
European Patent Office
Office européen des brevets

(11) **EP 4 541 905 A1**

(12) **EUROPEAN PATENT APPLICATION**
published in accordance with Art. 153(4) EPC

(43) Date of publication:
**23.04.2025 Bulletin 2025/17**

(21) Application number: **22946784.0**

(22) Date of filing: **14.06.2022**

(51) International Patent Classification (IPC):
***C12Q 1/6834*** (2018.01)      ***C12M 1/00*** (2006.01)

(52) Cooperative Patent Classification (CPC):
**C12M 1/00; C12Q 1/6834**

(86) International application number:
**PCT/JP2022/023851**

(87) International publication number:
**WO 2023/242964 (21.12.2023 Gazette 2023/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **HITACHI HIGH-TECH CORPORATION**
**Tokyo 105-6409 (JP)**

(72) Inventors:
• **FUJIOKA Michiru**
  **Tokyo 105-6409 (JP)**
• **AKIMOTO Kaoru**
  **Tokyo 105-6409 (JP)**
• **MATSUMOTO Yuki**
  **Tokyo 105-6409 (JP)**

(74) Representative: **MERH-IP Matias Erny Reichl Hoffmann**
**Patentanwälte PartG mbB**
**Paul-Heyse-Straße 29**
**80336 München (DE)**

(54) **NUCLEIC ACID ELUTION LIQUID AND NUCLEIC ACID ELUTION METHOD USING SAID NUCLEIC ACID ELUTION LIQUID**

(57)    The present invention improves the recovery rate of nucleic acid bonded to a nucleic acid capturing carrier, and recovers the nucleic acid at a high concentration. In the present invention, nucleic acid bonded to a nucleic acid capturing carrier is recovered by using a nucleic acid elution liquid containing: a polar solvent for dissolving the nucleic acid; and a hydrophobic solution that undergoes liquid-liquid layer separation with respect to the polar solvent.

[FIG. 1]

NUCLEIC ACID EXTRACTION STEP — STEP 1

↓

STEP FOR BINDING TO NUCLEIC ACID CAPTURING CARRIER — STEP 2

↓

WASHING STEP — STEP 3

↓

STEP FOR ELUTION FROM NUCLEIC ACID CAPTURING CARRIER — STEP 4

↓

END

EP 4 541 905 A1

**Description**

Technical Field

The present invention relates to a nucleic acid elution liquid to elute nucleic acid from a carrier that has absorbed the nucleic acid, and a nucleic acid elution method using the nucleic acid elution liquid.

Background Art

In recent years, information obtained through nucleic acid analysis such as cancer genome testing using a next-generation sequencing (NGS) system has been actively used in a variety of fields such as a medical field, a clinical testing field, and pharmaceutical and food industries. In this nucleic acid analysis, nucleic acid extraction from various biological samples such as blood, tissues, and cultured cells becomes an essential pretreatment step.

As a nucleic acid extraction method, instead of using harmful organic solvents such as phenol or chloroform, a method performed based on the properties of nucleic acid binding to silica in the presence of a chaotropic agent, and a method performed based on the properties of nucleic acid binding to silica in the presence of an organic solvent have been generally used. By using the above-mentioned methods, a nucleic acid extraction method using a nucleic acid capturing chip which incorporates a silica-containing solid phase as a nucleic acid capturing carrier, and a method using magnetic beads (nucleic acid capturing carriers) each having a surface covered with silica have been reported. Each of these methods includes a step of binding nucleic acid to a nucleic acid capturing carrier and an elution step of eluting the nucleic acid from the nucleic acid capturing carrier by using an elution liquid.

In the case of a method using the magnetic beads, after the elution step, the magnetic beads are recovered from the elution liquid by using a magnet. As an example, a method is performed in such a manner that an elution liquid containing magnetic beads is suctioned into a dispensing tip, the magnetic beads are held and stored in the dispensing tip by using a magnet, and only the elution liquid is discharged from the dispensing tip. Further, as another example, a method is performed in such a manner that a rod-shaped magnet (which may be covered) is inserted into an elution liquid containing magnetic beads so as to recover the magnetic beads from the elution liquid.

Further, Non-PTL 1 discloses a method of recovering nucleic acid from a silica membrane by causing an elution liquid to pass through the silica membrane that has absorbed the nucleic acid. In the method disclosed in Non-PTL 1, the elution liquid is caused to pass through the silica membrane using a method such as centrifugation or suction. In this case, in order to recover the elution liquid remaining in the silica membrane, mineral oil is caused to pass through the silica membrane using the same method such as centrifugation or suction. The elution liquid remaining in the silica membrane is reliably recovered by causing the mineral oil to pass through the silica membrane.

Furthermore, PTLs 1 to 3 disclose a system adapted to use magnetic beads disclosed in PTL 4 in order to elute nucleic acid captured on the magnetic beads. In the system disclosed in PTLs 1 to 3, an aqueous solution such as a washing liquid or an elution liquid is disposed in a column via a layer of oil, and the magnetic beads are caused to pass through the column so as to wash the magnetic beads and elute the nucleic acid from the magnetic beads. In other words, by providing the layer of oil in the column, an aqueous solution such as a washing liquid or an elution liquid is prevented from being mixed.

Citation List

Patent Literature

PTL 1: JP2017-184626A
PTL 2: JP2016-067274A
PTL 3: JP2014-093988A
PTL 4: JP2017-176023A

Non-Patent Literature

NON-PTL 1: npj Precision Oncology volume 4, Article number: 3 (2020)

Summary of Invention

Technical Problem

Meanwhile, when the nucleic acid is recovered using the nucleic acid capturing carrier such as magnetic beads as described above, it is preferable to use a system having excellent nucleic acid recovery efficiency. In particular, in a case where analysis is performed on trace amounts of nucleic acid contained in a biological sample, when nucleic acid recovery efficiency is low, there is a problem in that analysis using the recovered nucleic acid cannot be accurately performed. Meanwhile, in order to efficiently recover the nucleic acid captured by the nucleic acid capturing carrier, when the liquid volume of the elution liquid is increased, the concentration of the nucleic acid in the elution liquid becomes lower. In this case, there is also a problem in that a complicated step such as a step of concentrating nucleic acid is required before subsequent analysis is performed.

However, in the above-mentioned conventional system, in order to increase nucleic acid recovery efficiency from the nucleic acid capturing carrier, there is only one method of increasing the liquid volume of the elution liquid, but this method has a problem in that it is not possible to recover nucleic acid having a high concentration in the elution liquid. Therefore, in view of the above-mentioned circumstances, an object of the present invention is to provide a nucleic acid elution liquid and a nucleic acid elution method using the nucleic acid elution liquid that are capable of, when a nucleic acid capturing carrier is used to recover nucleic acid, achieving excellent nucleic acid recovery efficiency and recovering nucleic acid having a high concentration in the elution liquid.

Solution to Problem

In order to achieve the above-mentioned object, as a result of conducting intensive research by the present inventors, it was found that nucleic acid captured on a nucleic acid capturing carrier can be recovered at a high concentration in a polar solvent by using a mixed solvent containing the polar solvent capable of dissolving the nucleic acid and a hydrophobic solution capable of performing liquid-liquid phase separation between the hydrophobic solution and the polar solvent as a nucleic acid elution liquid for eluting the nucleic acid from the nucleic acid capturing carrier, thereby leading to completion of the present invention.

The present invention includes the following.

[1] A nucleic acid elution liquid adapted to elute nucleic acid bound to a nucleic acid capturing carrier, the nucleic acid elution liquid containing:

　　a polar solvent capable of dissolving the nucleic acid; and
　　a hydrophobic solution capable of performing liquid-liquid phase separation between the hydrophobic solution and the polar solvent.

[2] The nucleic acid elution liquid according to [1], in which the hydrophobic solution has a kinetic viscosity of 17 mm$^2$/s or less at 40°C.
[3] The nucleic acid elution liquid according to [1], in which the hydrophobic solution has a kinetic viscosity of 10 mm$^2$/s or less at 25°C.
[4] The nucleic acid elution liquid according to [1], in which the hydrophobic solution contains at least one component selected from a group consisting of silicone oil, fluorine-based oil, and liquid paraffin.
[5] A nucleic acid elution method including a step of causing a nucleic acid capturing carrier capturing nucleic acid and a nucleic acid elution liquid to contact each other and eluting the nucleic acid from the nucleic acid capturing carrier, in which the nucleic acid elution liquid contains:

　　a polar solvent capable of dissolving the nucleic acid; and
　　a hydrophobic solution capable of performing liquid-liquid phase separation between the hydrophobic solution and the polar solvent.

[6] The nucleic acid elution method according to [5], in which the hydrophobic solution contained in the nucleic acid elution liquid has a kinetic viscosity of 17 mm$^2$/s or less at 40°C.
[7] The nucleic acid elution method according to [5], in which the hydrophobic solution contained in the nucleic acid elution liquid has a kinetic viscosity of 10 mm$^2$/s or less at 25°C.
[8] The nucleic acid elution method according to [5], in which the hydrophobic solution contained in the nucleic acid elution liquid contains at least one component selected from a group consisting of silicone oil, fluorine-based oil, and liquid paraffin.
[9] The nucleic acid elution method according to [5], in which, after the step of causing the nucleic acid capturing carrier and the nucleic acid elution liquid to contact each other, the nucleic acid capturing carrier is separated from the nucleic acid elution liquid, and the nucleic acid is recovered in the polar solvent contained in the nucleic acid elution liquid.

[10] The nucleic acid elution method according to [5], further including a step of causing a biological sample prepared from an organism to be examined and the nucleic acid capturing carrier to contact each other and capturing nucleic acid derived from the biological sample on the nucleic acid capturing carrier.

[11] A nucleic acid treatment apparatus including:

a reaction vessel adapted to cause a nucleic acid capturing carrier capturing nucleic acid and a nucleic acid elution liquid to contact each other; and

a separation device adapted to separate any one of the nucleic acid capturing carrier and the nucleic acid elution liquid from the reaction vessel, in which the nucleic acid elution liquid contains:

a polar solvent capable of dissolving the nucleic acid; and

a hydrophobic solution capable of performing liquid-liquid phase separation between the hydrophobic solution and the polar solvent.

[12] The nucleic acid treatment apparatus according to [11], in which:

the nucleic acid capturing carrier is a magnetic bead,

the separation device includes a magnetic body part containing a magnetic material, and

the separation device attracts the nucleic acid capturing carrier to the magnetic body part and separates the nucleic acid capturing carrier from the nucleic acid elution liquid in the vessel.

Advantageous Effects of Invention

With a nucleic acid elution liquid according to the present invention, when nucleic acid is eluted from a nucleic acid capturing carrier, the liquid volume can be increased by a hydrophobic solution, and the nucleic acid captured by the nucleic acid capturing carrier can be recovered in a polar solvent at a high concentration.

In addition, a nucleic acid elution method according to the present invention uses a nucleic acid elution liquid containing a polar solvent capable of dissolving nucleic acid and a hydrophobic solution. Accordingly, when nucleic acid is eluted from a nucleic acid capturing carrier, the liquid volume can be increased by the hydrophobic solution, and the nucleic acid captured by the nucleic acid capturing carrier can be recovered in the polar solvent at a high concentration.

Furthermore, a nucleic acid treatment apparatus according to the present invention uses a nucleic acid elution liquid containing a polar solvent capable of dissolving nucleic acid and a hydrophobic solution. Accordingly, when nucleic acid is eluted from a nucleic acid capturing carrier, the liquid volume of the nucleic acid elution liquid in a reaction vessel can be increased, and the nucleic acid captured by the nucleic acid capturing carrier can be recovered in the polar solvent at a high concentration.

Brief Description of Drawings

[Fig. 1] Fig. 1 is a flowchart showing an example of a nucleic acid extraction method to which the present invention is applied.

[Fig. 2] Fig. 2 is a schematic diagram showing an example of a system adapted to extract nucleic acid using magnetic particles.

[Fig. 3] Fig. 3 is a schematic diagram showing another example of the system adapted to extract nucleic acid using magnetic particles.

[Fig. 4] Fig. 4 is a characteristic diagram showing a relationship between the liquid volume of a nucleic acid elution liquid and a DNA recovery rate.

[Fig. 5] Fig. 5 is a characteristic diagram showing a relationship between the liquid volume of a nucleic acid elution liquid and a recovery rate of the nucleic acid elution liquid.

[Fig. 6] Fig. 6 is a characteristic diagram showing a relationship between composition conditions of the nucleic acid elution liquid and the DNA recovery rate.

[Fig. 7] Fig. 7 is a characteristic diagram showing a relationship between the amount of hydrophobic solution added to the nucleic acid elution liquid and the DNA recovery rate.

[Fig. 8] Fig. 8 is a characteristic diagram showing a relationship between the amount of hydrophobic solution added to the nucleic acid elution liquid and the recovery rate of the nucleic acid elution liquid.

Description of Embodiments

Hereinafter, an embodiment of the present invention will be described in detail with reference to the drawings.

A nucleic acid elution liquid according to the present invention contains a polar solvent capable of dissolving nucleic acid and a hydrophobic solution capable of performing liquid-liquid phase separation between the hydrophobic solution and the polar solvent. The nucleic acid elution liquid can be brought into contact with a nucleic acid capturing carrier that has captured the nucleic acid, thereby making it possible to elute the nucleic acid from the nucleic acid capturing carrier. By using the nucleic acid elution liquid according to the present invention, the recovery rate of the nucleic acid from the nucleic acid capturing carrier (nucleic acid recovery rate) can be significantly improved.

The polar solvent is a solvent having a polarity capable of dissolving hydrophilic nucleic acid and is also referred to as a highly polar solvent. As the polar solvent, an aqueous solution having a low salt concentration or pure water can be typically used. As an example, a solvent consisting of 10 mmol/L Tris (pH 8.5) and 0.1 mmol/L EDTA can be used as the polar solvent.

The hydrophobic solution is a solution that is mainly composed of non-polar molecules and has a property of performing "liquid-liquid phase separation" between the hydrophobic solution and the polar solvent. Therefore, the nucleic acid elution liquid containing the hydrophobic solution and the polar solvent is in a state in which a layer containing the hydrophobic solution and a layer containing the polar solvent are separated from each other. Although the composition of the hydrophobic solution is not particularly limited, oil, that is, a hydrocarbon compound, is used as a main component. An example of such a hydrophobic solution may include a liquid containing a type of oil selected from the group consisting of silicone oil, fluorine-based oil, and liquid paraffin. It is noted that the hydrophobic solution is not limited to a solution consisting of a single composition, and may be a mixed solution consisting of a plurality of compositions. As silicone oil, dimethyl silicone oil, methyl hydrogen silicone oil, methyl phenyl silicone oil, cyclic dimethyl silicone oil, and the like can be used.

In addition, in the case of a hydrophobic solution, the kinetic viscosity at 40°C is preferably 17 mm$^2$/s or less and is more preferably 14 mm$^2$/s or less, and the kinetic viscosity at 25°C is most preferably 10 mm$^2$/s or less. When the hydrophobic solution has the kinetic viscosity of 17 mm$^2$/s or less at 40°C, 14 mm$^2$/s or less at 40°C, or 10 mm$^2$/s or less at 25°C, the nucleic acid recovery rate can be further improved. The kinetic viscosity of the hydrophobic solution is defined as a numerical value at a predetermined temperature, and the kinetic viscosity tends to decrease as the temperature increases. Therefore, the temperature at which the nucleic acid elution liquid is used is set to 40°C or higher, thereby making it possible to achieve an excellent nucleic acid recovery rate even when a hydrophobic solution having a kinetic viscosity exceeding 17 mm$^2$/s at 40°C is used.

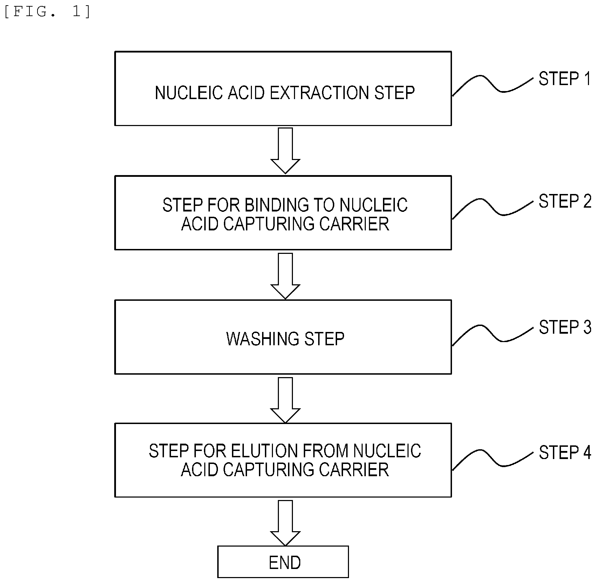

The nucleic acid elution liquid constituted as described above can be used to elute nucleic acid from a nucleic acid-binding nucleic acid capturing carrier. For example, the nucleic acid elution liquid according to the present invention can be applied to a nucleic acid extraction method of a flowchart shown in Fig. 1. It is noted that the nucleic acid elution liquid according to the present invention is used in step 4: a step for elution from a nucleic acid capturing carrier in the flowchart shown in Fig. 1. It is noted that the application of the nucleic acid elution liquid according to the present invention is not limited to the flowchart shown in Fig. 1.

In the present nucleic acid extraction method, first, in step 1, a dissolving reagent for adjusting a sample containing nucleic acid and a binding reagent for binding the nucleic acid to a nucleic acid binding carrier are prepared, and the dissolving reagent and the binding reagent are added to a biological sample such as an animal cell. It is noted that the dissolving reagent and the binding reagent may be prepared as a single liquid and may be added to the biological sample. In step 1, the nucleic acid contained in the biological sample is dissolved in the solution, thereby making it possible to prepare a sample containing the nucleic acid. Here, the nucleic acid means deoxyribonucleic acid and ribonucleic acid having a doublestranded or single-stranded structure or a partially doublestranded or single-stranded structure. In the nucleic acid extraction method, the biological sample is not limited to animal cells, and microbial cells such as bacteria or fungi, plant cells, or viruses may be used as the biological sample. Particularly, biological samples such as whole blood, plasma, serum, sputum, urine, cultured cells, and cultured bacteria are preferably used as biological samples.

It is noted that, in step 1, techniques such as heating and stirring can be appropriately applied to promote dissolution of the biological sample. In addition, after the biological sample is dissolved by the dissolving reagent, a solid content may be removed by centrifugation operation.

Examples of a substance that promotes binding of nucleic acid to a nucleic acid-binding solid phase and is contained in the above-mentioned binding reagent can include chaotropic agents such as NaI (sodium iodide), KI (potassium iodide), NaClO4 (sodium perchlorate), NaSCN (sodium thiocyanate), GuSCN (guanidine thiocyanate), and GuHCl (guanidine hydrochloride). The binding reagent is not limited thereto, and any substance that promotes binding of nucleic acid to a nucleic acid-binding solid phase may be used.

Next, in step 2 of the present nucleic acid extraction method, a nucleic acid capturing carrier is brought into contact with the sample containing the nucleic acid prepared in step 1, and the nucleic acid is bound to the nucleic acid binding carrier. Examples of the nucleic acid capturing carrier include glass particles, silica particles, silica-coated magnetic particles, quartz filter paper, quartz wool or crushed products thereof, and diatomaceous earth. In other words, a substance containing silicon oxide or an organic polymer having a hydroxyl group on the surface thereof can be used as the nucleic acid capturing carrier. The nucleic acid capturing carrier is not limited thereto, and any substance that captures nucleic acid may be used.

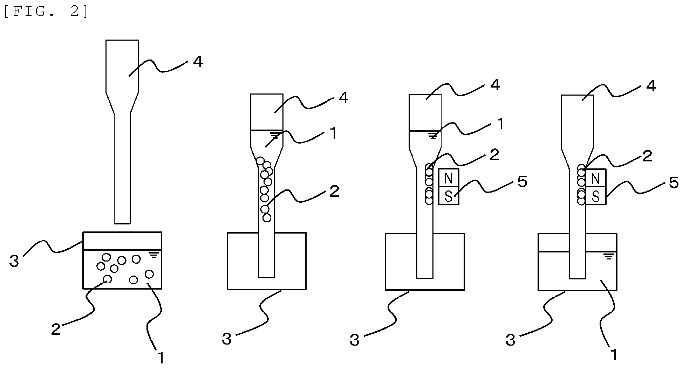

In particular, the present invention can be applied to a method of extracting nucleic acid using silica-coated magnetic particles (also called magnetic beads). Specifically, as shown in Fig. 2, first, a sample 1 containing nucleic acid prepared in step 1 is mixed with a plurality of magnetic particles 2 in a reaction vessel 3. Accordingly, it is possible to allow the nucleic acid contained in the sample 1 to bind to the magnetic particles 2. At this time, the sample 1 can be effectively brought into contact with the magnetic particles 2 by suctioning and discharging the magnetic particles 2 together with the sample 1 using a dispensing tip 4. In this case, a pipetter, a syringe, a pump, or the like is directly or indirectly attached to the dispensing tip 4 in an airtight state, thereby enabling the sample 1 to be suctioned into the dispensing tip 4 or discharged therefrom by vacuum decompression, pressurization, centrifugation, and the like using the pipetter, the syringe, and the pump.

Then, as shown in Fig. 2, in a state in which the magnetic particles 2 are suctioned into the dispensing tip 4 together with the sample 1, a magnetic body 5 is brought close to the side surface of the dispensing tip 4. As a result, the magnetic particles 2 in the dispensing tip 4 are captured by the inner wall of the dispensing tip 4. In this state, the sample 1 in the dispensing tip 4 is discharged into the reaction vessel 3, thereby making it possible to separate the nucleic acid-binding magnetic particles 2 from the sample 1.

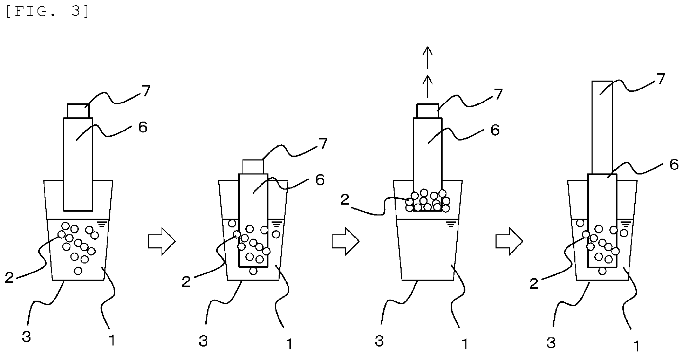

As another example, as shown in Fig. 3, first, a plurality of magnetic particles 2 are mixed with the sample 1 containing the nucleic acid prepared in step 1 in the reaction vessel 3. Accordingly, it is possible to allow the nucleic acid contained in the sample 1 to bind to the magnetic particles 2. In this case, the sample 1 containing the magnetic particles 2 is stirred so as to enable the sample 1 to effectively contact the magnetic particles 2.

Thereafter, as shown in Fig. 3, the magnetic particles 2 in the sample 1 can be captured on the bottom surface and the side surfaces of a cover 6 by immersing a magnetic rod 7 in the reaction vessel 3 in a state of inserting the magnetic rod 7 into the cylindrical cover 6. Then, the nucleic acid-binding magnetic particles 2 can be separated from the sample 1 by removing the magnetic rod 7 from the reaction vessel 3 in a state in which the magnetic rod 7 is inserted into the cylindrical cover 6.

In addition, the magnetic rod 7 is removed from the cover 6 in a state in which the magnetic particles 2 in the sample 1 are captured on the bottom surface and the side surfaces of the cover 6, thereby enabling the magnetic particles 2 captured on the bottom surface and the side surfaces of the cover 6 to be returned to the sample 1. In this manner, the magnetic particles 2 in the sample 1 are captured on the bottom surface and the side surfaces of the cover 6, and then the operation of returning the magnetic particles 2 captured on the bottom surface and the side surfaces of the cover 6 to the sample 1 is repeatedly performed, thereby enabling the sample 1 to be effectively brought into contact with the magnetic particles 2.

Although not shown in the drawing, the nucleic acid capturing carrier may be arranged inside a dispensing tip, and a solution containing nucleic acid is suctioned into the dispensing tip, thereby allowing the nucleic acid capturing carrier to bind to the nucleic acid in the solution. In such a case, suction and discharge of the solution is repeatedly performed, thereby enabling the solution to effectively contact the nucleic acid capturing carrier arranged inside the dispensing tip. In addition, although not shown in the drawing, a nucleic acid capturing column filled with a nucleic acid capturing carrier can also be used. In this case, a sample containing nucleic acid is added to an upper portion of the column, and the sample passes through the column by pressurization, centrifugation, vacuum decompression, and the like, thereby enabling the nucleic acid capturing carrier and the sample to contact each other.

In step 3 of the nucleic acid extraction method, the nucleic acid-binding nucleic acid capturing carrier is washed, and non-specifically bound substances are removed from the nucleic acid capturing carrier. Although a washing reagent is not particularly limited, for example, any washing reagent may be used as long as it can remove the reagent added in step 1 and impurities from the nucleic acid capturing carrier while maintaining the binding of the nucleic acid to the nucleic acid capturing carrier. As the washing reagent, an organic compound such as low alcohol or low molecular ketone can be used. As the washing reagent, for example, ethanol, isopropanol, and the like can be used. Particularly, it is preferable to use ethanol with a concentration of 70% or more.

In addition, although a technique for bringing the washing reagent into contact with the nucleic acid capturing carrier is not particularly limited, a technique (for example, the method shown in Figs. 2 and 3) used when the sample containing the nucleic acid is brought into contact with the nucleic acid capturing carrier can be appropriately applied.

In step 4 of the nucleic acid extraction method, nucleic acid bound to the nucleic acid capturing carrier is eluted from the nucleic acid capturing carrier. In detail, the nucleic acid is recovered in the nucleic acid elution liquid by bringing the nucleic acid elution liquid into contact with the nucleic acid-binding nucleic acid capturing carrier. Although a technique for bringing the nucleic acid elution liquid into contact with the nucleic acid capturing carrier is not particularly limited, a technique (for example, the method shown in Figs. 2 and 3) used when the sample containing the nucleic acid is brought into contact with the nucleic acid capturing carrier can be appropriately applied.

The nucleic acid elution liquid according to the present invention contains a polar solvent capable of dissolving nucleic acid and a hydrophobic solution, in which the nucleic acid is dissolved in the polar solvent. Therefore, by using the nucleic acid elution liquid according to the present invention, it is possible to elute nucleic acid having a high concentration in the polar solvent.

For example, when the nucleic acid capturing carrier is brought into contact with the nucleic acid elution liquid according to the method shown in Figs. 2 and 3, it is difficult to elute all or most of the nucleic acids from the nucleic acid capturing carrier unless the entire nucleic acid capturing carrier such as the magnetic particles 2 is brought into contact with the nucleic acid elution liquid. Further, even in methods other than the method shown in Figs. 2 and 3, it is important that the nucleic acid elution liquid is reliably brought into contact with the nucleic acid capturing carrier in order to elute all or most of the nucleic acids from the nucleic acid capturing carrier.

In the method shown in Fig. 2, when the nucleic acid elution liquid volume is small, it is preferable to reduce the inner diameter of the dispensing tip depending on the volume of liquid to be suctioned. However, when the inner diameter is too small, the nucleic acid capturing carrier may clog the dispensing tip, so it is preferable to ensure a certain size of the inner diameter of the dispensing tip. Therefore, when the inner diameter is constantly maintained, it is preferable to limit the volume of nucleic acid elution liquid that can be suctioned through the dispensing tip.

The same applies to the method shown in Fig. 3. It is preferable to fill the magnetic rod with the nucleic acid elution liquid up to the height at which the nucleic acid capturing carrier is covered with the nucleic acid elution liquid, it is preferable to secure a certain level of liquid volume, and it is preferable to limit the nucleic acid elution liquid volume.

The nucleic acid elution liquid according to the present invention is stirred in a state of containing the hydrophobic solution, so that the polar solvent can be suppressed to a small amount even if a liquid volume to be surely brought into contact with the nucleic acid capturing carrier is used. In other words, by using the nucleic acid elution liquid according to the present invention, the nucleic acid elution liquid can be surely brought into contact with the nucleic acid capturing carrier so as to elute all or most of the nucleic acid from the nucleic acid capturing carrier. Additionally, nucleic acid having a high concentration can be dissolved in the polar solvent.

In this manner, by using the nucleic acid elution liquid according to the present invention, it is possible to increase the nucleic acid recovery rate from the nucleic acid-binding nucleic acid capturing carrier, and to obtain a highly concentrated nucleic acid solution. It is noted that, in the obtained nucleic acid solution, a layer of the hydrophobic solution and a layer of the polar solvent containing nucleic acid can be separated from each other, and the layer of the polar solvent can be used for subsequent analysis, and two layers including the layer of the hydrophobic solution layer and the layer of the polar solvent containing nucleic acid can also be used for subsequent analysis.

Analysis using the obtained nucleic acid solution is not specifically limited, and examples thereof can include sequence analysis using a next-generation sequencer, electrophoresis, high-performance liquid chromatography-mass spectrometry (LC-MS), and the like. When these specific analysis methods are performed, the two layers including the layer of the hydrophobic solution and the layer of the polar solvent containing nucleic acid can be used.

Examples

Hereinafter, the present invention will be described in more detail using examples, but the technical scope of the present invention is not limited to the following examples.

[Example 1]

1. Object

In this example, a relationship between nucleic acid concentration, the nucleic acid elution liquid volume, and extraction efficiency was investigated, and then a concentration method of extracting nucleic acid with a small liquid volume and a high concentration was examined. Specifically, a mixture obtained by adding a hydrophobic solution (oil) to a polar solvent (main component: water) was used as a nucleic acid elution liquid. A property indicating that DNA can be dissolved in the polar solvent, but DNA is not dissolved in the hydrophobic solution (oil) was utilized.

An object of this example was to elute highly concentrated DNA using a smaller liquid volume (less than 50 $\mu$L) than before by using a nucleic acid elution liquid containing a hydrophobic solution. It is noted that it was considered that, if the DNA recovery rate remained the same as before, DNA was more highly concentrated in the polar solvent by addition of the hydrophobic solution than a conventional one.

2. Target

In this example, as an example, a nucleic acid preparation system compatible with liquid biopsy was used, and cell-free DNA (hereinafter referred to as cfDNA) in blood was a target of the study.

3. Materials

3-1. Reagents

Table 1 shows a kinetic viscosity and a flash point (listed in the catalog and SDS) of each of the hydrophobic solutions (oil) used in a nucleic acid elution liquid. Hydrophobic solutions (oils) A, B, and D are each silicone oil having a dimethylpolysiloxane structure. Further, the hydrophobic solutions (oils) A, B, and D are extremely chemically inert and are characterized by excellent heat resistance, cold resistance, and viscosity stability, and have a variety of viscosity lineups. Hydrophobic solutions (oil) C and E (mineral oil) are hydrophobic and low-viscosity oils. Further, the hydrophobic solutions C and E are added to a reaction solution for the polymerase chain reaction (PCR) so as to prevent evaporation of the reaction solution during PCR and do not inhibit the PCR process after nucleic acid extraction.

[Table 1]

| Hydrophobic solution (oil) | A | B | C | D | E |
|---|---|---|---|---|---|
| kinetic viscosity mm$^2$/s () is measured temperature | 2 (25°C) | 5 (25°C) | 5 (temperature description) | 10 (25°C) | 14 to 17 (38°C) |
| Flash point °C () is testing method | 88 (closed type) | 102 (opened type) > 61 (closed type) | > 101.1 (closed type) | 176 (opened type) > 94 (closed type) | > 160 (opened type) |

3-2. Simulated plasma sample

A PCR product having a base length of 140 bp was added as simulated cfDNA (hereafter referred to as simulated cfDNA) to human plasma that does not contain cfDNA or DNA. The concentration of the simulated cfDNA added was adjusted depending on each experiment.

4. Method

4-1. cfDNA extraction method

4-1-1. cfDNA extraction method 1

Simulated cfDNA was extracted. An example of an extraction method applied in this example is shown in Fig. 3. That is, first, a reagent that solubilizes proteins in the blood is added, and DNA in a sample is bound to magnetic beads. The mixture is stirred by upward-and-downward movement of a rod-shaped plastic comb, and when the comb is immersed in the solution in a state in which a magnet is set inside the comb, the DNA-binding magnetic beads are adsorbed to a tip portion of the comb. Next, in a state in which the magnet is still set inside the comb, the comb is moved to a washing liquid with the magnetic beads still attached thereto. When only the comb is left in the washing liquid and the magnet is pulled up, the magnetic beads are released into the washing liquid. Similarly, stirring, adsorption of the magnetic beads, and movement of the magnetic beads are repeatedly performed, and washing is performed a plurality of times using the washing liquid. Next, the magnetic beads are moved to a nucleic acid elution liquid, and the DNA bound to the magnetic beads is eluted in the nucleic acid elution liquid. After the DNA has been released from the magnetic beads, the magnetic beads are adsorbed to the magnet via the comb and are separated from the nucleic acid elution liquid.

Next, the specific procedures for cfDNA extraction are described below. First, two 24-deep-well plates were prepared, and each of the reagents was dispensed into a corresponding one of the designated positions on the plates according to Table 2.

[Table 2]

|  | Position | Reagent | Liquid Volume |
|---|---|---|---|
| Plate 1 | A | Dissolving binding reagent | 3 mL |
|  |  | Magnetic bead reagent | 37 μL |
|  | B | Dissolving binding reagent | 3 mL |
|  |  | Magnetic bead reagent | 37 μL |
|  | C | Washing reagent | 1 mL |
|  | D | Washing reagent | 1 mL |
| Plate 2 | A | Nucleic acid elution liquid | Any liquid volume |
|  | B | Ethanol of 80% | 500 μL |
|  | C | Ethanol of 80% | 2 mL |
|  | D | Tip comb (Device consumables) | - |

Next, 5 mL of the simulated plasma sample was divided and added to rows A and B of the plate 1 and was gently mixed by pipetting. Thereafter, nucleic acid extraction was performed. After nucleic acid extraction was completely performed, DNA extracted in the row A of the plate 2 was immediately recovered in a 500 μL tube for storage.

4-1-2. cfDNA extraction method 2

cfDNA extraction was also performed using a different method. Specific procedures thereof are described below.
A reagent was mixed with 1 mL of the simulated plasma sample, as shown in Table 3. When a plurality of samples were used, batch processing was performed thereon.

[Table 3]

| Reagent | Composition |
|---|---|
| 20 mg/mL Proteinase K | 15 μL |
| Simulated plasma sample | 1 mL |
| 20% SDS | 50 μL |
| Total | 1.065 mL |

Then, the sample was incubated at 60°C for 20 minutes. During this processing, the sample was removed from the incubator every five minutes and was stirred for one minute. Then, the sample was left to stand on the ice for five minutes. Finally, the sample was mixed with a magnetic bead-containing buffer, as shown in Table 4. When a plurality of samples were used, batch processing was performed thereon.

[Table 4]

| Reagent | Composition |
|---|---|
| Dissolving binding reagent | 1.25 mL |
| Magnetic bead reagent | 15 μL |
| Sample | 1.065 mL |
| Total | 2.33 mL |

After sufficiently mixing the solution in the tube, the tube was left to stand on the magnet for five minutes. A supernatant was completely removed from the tube in this state. The tube was removed from the magnet, 1 mL of dissolving binding reagent was added per 1 mL of sample to a magnetic bead-containing tube and was stirred. Thereafter, the sample was transferred to a new 1.5 mL tube. The tube was fixed to the magnet again, the recovered supernatant was returned to the original tube, and the remaining magnetic beads were further recovered and were added to the previous 1.5 mL tube. The supernatant was completely removed in a state in which the tube was fixed to the magnet. The tube was removed from the magnet, 1 mL of dissolving binding reagent was added to the tube, and the tube was stirred for 30 seconds. The stirred sample was left to stand on the magnet for two minutes or until the color of the supernatant became transparent. The supernatant was completely removed in a state in which the tube was fixed to the magnet. The tube was removed from the magnet, 1 mL of 80% ethanol was added to the tube, and the tube was stirred for 30 seconds. The stirred sample was left to stand on the magnet for two minutes or until the color of the supernatant became transparent. The supernatant was completely removed in a state in which the tube was fixed to the magnet.

The tube was left to stand for three to five minutes in a state of being fixed to the magnet and was dried. Thereafter, the remaining liquid was completely removed from the tube. The tube was removed from the magnet, and a dissolving binding reagent, which is a polar solvent, was added to the tube. Further, a hydrophobic solution (oil) was added to the tube depending on the experimental conditions. The liquid volume varied depending on the experimental conditions.

Then, the tube was stirred for five minutes. The stirred sample was left to stand on the magnet for two minutes or until the color of the supernatant became transparent, and the supernatant was recovered from the sample.

4-1-3. Evaluation of nucleic acid elution liquid

The nucleic acid elution liquid volume and DNA concentration were obtained, and the DNA recovery rate, which is synonymous with the nucleic acid extraction efficiency, was calculated.

Regarding the nucleic acid elution liquid volume, the weight of an empty tube and the weight of the tube after nucleic acid elution liquid recovery were respectively measured, and the nucleic acid elution liquid volume was calculated from a difference between the measured weights of the respective tubes. A liquid volume of simulated cfDNA added to the simulated plasma sample was the volume of liquid taken with a pipette. The recovery rate of the nucleic acid elution liquid volume was calculated according to the following mathematical formula.

[Mathematical Formula 1]

$$\textit{Recovery rate of nucleic acid elution liquid amount (\%)} =$$

$$\frac{\textit{Nucleic acid elution liquid volume } (\mu L) \times 100}{\textit{Nucleic acid elution liquid volume used in experiment (hydrophobic solution is not included)}(\mu L)}$$

Regarding the DNA concentration, the DNA concentration used in the experiment and the DNA concentration in the nucleic acid elution liquid were measured by a fluorescent photometer. The experiment was performed in the same manner for a simulated plasma sample to which the simulated cfDNA was not added, and the measured value was subtracted as a background.

Regarding the DNA recovery rate, the amount of DNA was calculated by multiplying the liquid volume by the DNA concentration. Thereafter, the DNA recovery rate was calculated according to the following mathematical formula.

[Mathematical Formula 2]

$$\textit{Recovery rate of DNA (\%)} =$$

$$\frac{\textit{DNA amount in nucleic acid elution liquid } (ng) \times 100}{\textit{DNA amount in stimulated plasma sample used in experiment } (ng)}$$

5. Effect of nucleic acid elution liquid volume

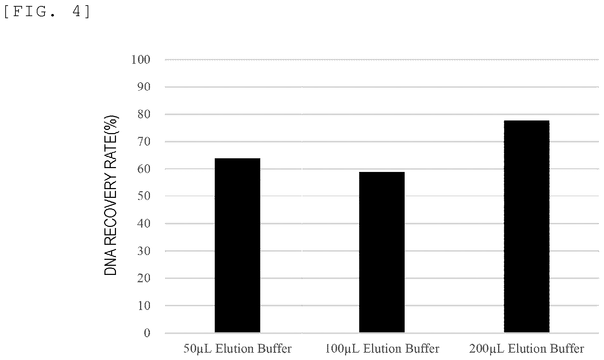

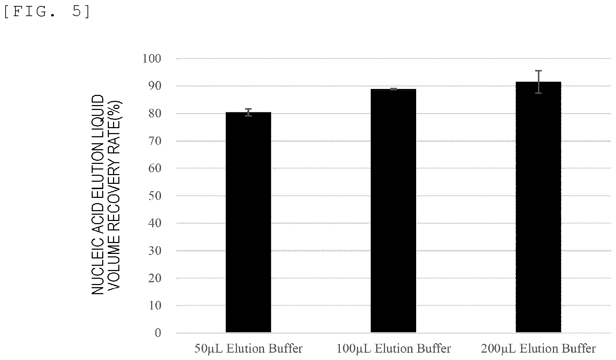

A simulated plasma sample obtained by adding simulated cfDNA thereto so as to form 15 ng/5 mL plasma was treated using a nucleic acid elution apparatus, and DNA was eluted under three conditions of 50, 100, and 200 μL of the nucleic acid elution liquid volume. Fig. 4 shows the DNA recovery rate for each liquid volume of the nucleic acid elution liquid. The measured DNA recovery rates for 50 μL and 100 μL were 63.8% and 58.9%, respectively, and no significant difference therebetween was observed. On the other hand, the DNA recovery rate for 200 μL was increased to 77.7%. Fig. 5 shows the recovery rate for the nucleic acid elution liquid volume. When the nucleic acid elution liquid volume was set to 50, 100, or 200 μL in the method shown in Fig. 3, the recovery rate was 80.4 to 91.5%, which was lower than the expected liquid volume. In addition, as the nucleic acid elution liquid volume increased, the recovery rate of the nucleic acid elution liquid increased. This indicates that the percentage of the nucleic acid elution liquid remaining on the magnetic beads increases as the nucleic acid elution liquid volume decreases.

6. Effect of hydrophobic solution in nucleic acid elution liquid

6-1. Comparison of kinetic viscosities of hydrophobic solutions (oils)

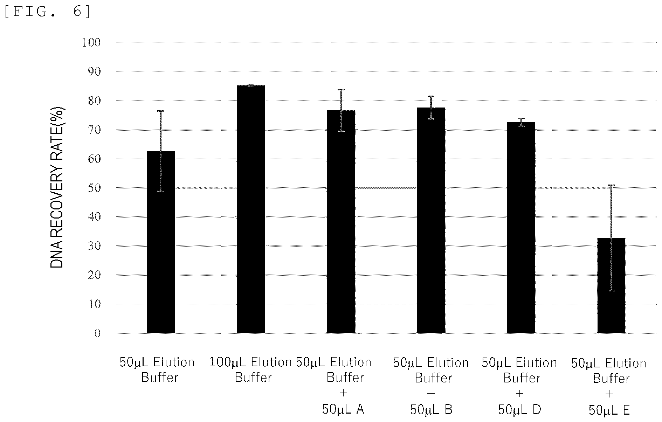

Elution of nucleic acids was manually performed from 1 mL of a simulated plasma sample obtained by adding simulated cfDNA thereto so as to form 50 to 120 ng/1 mL plasma. Regarding the nucleic acid elution liquid, a nucleic acid elution liquid obtained by adding 50 μL or 100 μL of a polar solvent thereto, and a nucleic acid elution liquid obtained by adding 50 μL of hydrophobic solution (oil) to 50 μL of a polar solvent were used. Four types of hydrophobic solutions (oils) having different kinetic viscosities were compared with each other, excluding C in Table 1. Fig. 6 shows the DNA recovery rate. As shown in Fig. 6, the average DNA recovery rate for 50 μL of nucleic acid elution liquid to which no hydrophobic solution (oil) was added was 62.7%. In contrast, the DNA recovery rates for the nucleic acid elution liquids to which hydrophobic solutions (oils: A, B, and D) each having a kinetic viscosity of 2 to 10 mm$^2$/s (25°C) were respectively added were 72.6 to 77.6%, which means that the DNA recovery rate for the nucleic acid elution liquid to which the hydrophobic solution (oil) was added was either unchanged or improved as compared with the DNA recovery rate for the nucleic acid elution liquid to which the hydrophobic solution (oil) was not added. On the other hand, when oil E having a kinetic viscosity of 14 to 17 mm$^2$/s (38°C) was added to the nucleic acid elution liquid, the average DNA recovery rate was 32.8%, which apparently shows deterioration in DNA recovery rate.

The experimental conditions under which the DNA recovery rate was not improved or was reduced may have been due to insufficient mixing conditions between the hydrophobic solution (oil) and the polar solvent. Accordingly, it is considered to improve the DNA recovery rate by optimizing these mixing conditions therebetween.

6-2. Comparison of addition amounts of hydrophobic solution (oil)

Nucleic acid extraction was performed using the 4-1-2. cfDNA extraction method 2 on 1 mL of a human plasma sample obtained by adding simulated cfDNA thereto so as to form approximately 0.75 ng/1 mL of plasma. Regarding the nucleic acid elution liquids, a nucleic acid elution liquid to which only 50 μL of polar solvent was added, a nucleic acid elution liquid to which 50 μL of polar solvent and 50 or 150 μL of hydrophobic solution (oil) were added, a nucleic acid elution liquid to which only 15 μL of polar solvent was added, and a nucleic acid elution liquid to which 15 μL of only polar solvent and 35, 85, or 185 μL of hydrophobic solution (oil) were added were used. The used hydrophobic solutions (oils) were C and B, both of which have a kinetic viscosity of 5 mm$^2$/s (C does not have the measured temperature described in SDS, and B is 25°C). From the above-described examination, "B" was selected because "B" has a low viscosity and a relatively high flash point (102°C) .

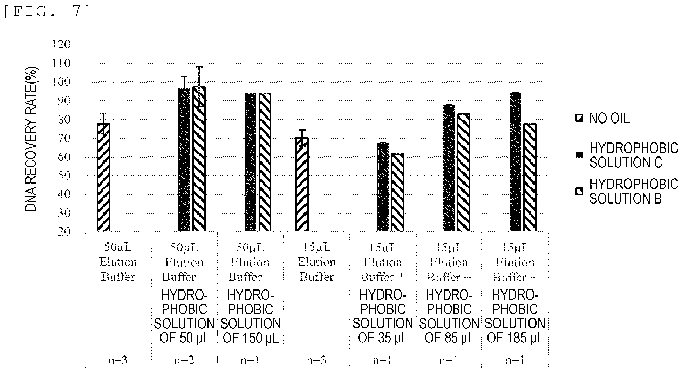

Fig. 7 shows the DNA recovery rates under respective conditions. In the case of a nucleic acid elution liquid to which only 50 μL of polar solvent was added, the average DNA recovery rate was 77.7%. In the case of a nucleic acid elution liquid to which 50 μL of polar solvent and an additional hydrophobic solution (oil) were added, the DNA recovery rate was improved to 93.7 to 97.4%. No difference was observed depending on the type of hydrophobic solution (oil). In the case of a nucleic acid elution liquid to which only 15 μL of polar solvent was added, the average DNA recovery rate was 70.1%. In the case of nucleic acid elution liquids respectively obtained by adding 85 μL of hydrophobic solution (oil) to the nucleic acid elution liquid to which only 15 μL of polar solvent was added and by adding 185 μL of hydrophobic solution (oil) thereto, the DNA recovery rates were respectively 82.8 to 87.7% and 77.8 to 94.2%, which showed that the DNA recovery rate of the nucleic acid elution liquid to which the hydrophobic solution (oil) was added was more improved than that of the nucleic acid elution liquid to which the hydrophobic solution (oil) was not added. The oil type C had a higher recovery rate than that of the oil type B.

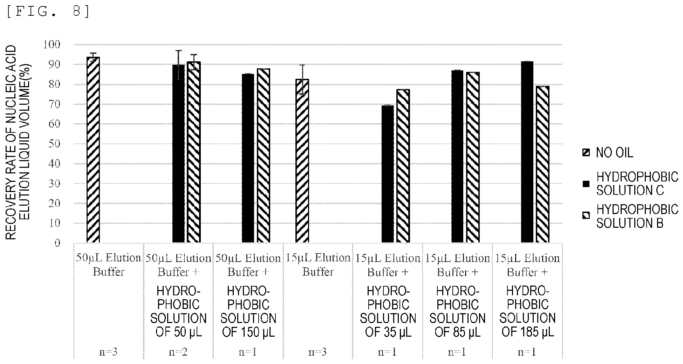

Fig. 8 shows the recovery rate of the nucleic acid elution liquid when elution was performed under each condition. In all the conditions, the recovery rate of each of the nucleic acid elution liquids was 69.3 to 93.5%, which was lower than the expected amount of the nucleic acid elution liquid of 15 μL or 50 μL. When the amount of nucleic acid elution liquid was 15 pL, the recovery rate of the nucleic acid elution liquid tended to increase by adding the hydrophobic solution C to the nucleic acid elution liquid. Accordingly, the DNA recovery rate also tended to increase. This indicates a reduction in the amount of nucleic acid elution liquid remaining on magnetic beads and the like.

7. Examination of results

The above-described results of the present examples apparently showed that, by using a nucleic acid elution liquid containing a polar solvent capable of dissolving nucleic acid and a hydrophobic solution, it is possible to elute the nucleic acid at a high concentration even if a small liquid volume of polar solvent is added (for example, less than 50 pL). Although it was found that a preferable kinetic viscosity of a hydrophobic solution is preferably in the range of 10 mm$^2$/s or less, it was suggested that, even if a kinetic viscosity of a hydrophobic solution is out of the preferable kinetic viscosity range, nucleic acid can be eluted at a high concentration by considering the mixing conditions with the polar solvent.

Regarding the addition amount of hydrophobic solution, the DNA recovery rate tended to increase when the addition amount of hydrophobic solution increased. However, it was suggested that, even if the liquid volume of hydrophobic solution is small, it is possible to elute nucleic acid at a high concentration by reducing evaporation during operation and remaining substances produced when magnetic beads and a nucleic acid elution liquid are separated from each other.

**Claims**

1.   A nucleic acid elution liquid adapted to elute nucleic acid bound to a nucleic acid capturing carrier, the nucleic acid elution liquid containing:

   a polar solvent capable of dissolving the nucleic acid; and
   a hydrophobic solution capable of performing liquid-liquid phase separation between the hydrophobic solution and the polar solvent.

2.   The nucleic acid elution liquid according to claim 1, wherein the hydrophobic solution has a kinetic viscosity of 17 $mm^2$/s or less at 40°C.

3.   The nucleic acid elution liquid according to claim 1, wherein the hydrophobic solution has a kinetic viscosity of 10 $mm^2$/s or less at 25°C.

4.   The nucleic acid elution liquid according to claim 1, wherein the hydrophobic solution contains at least one component selected from a group consisting of silicone oil, fluorine-based oil, and liquid paraffin.

5.   A nucleic acid elution method comprising a step of causing a nucleic acid capturing carrier capturing nucleic acid and a nucleic acid elution liquid to contact each other and eluting the nucleic acid from the nucleic acid capturing carrier,

   wherein the nucleic acid elution liquid contains:
   a polar solvent capable of dissolving the nucleic acid; and
   a hydrophobic solution capable of performing liquid-liquid phase separation between the hydrophobic solution and the polar solvent.

6.   The nucleic acid elution method according to claim 5, wherein the hydrophobic solution contained in the nucleic acid elution liquid has a kinetic viscosity of 17 $mm^2$/s or less at 40°C.

7.   The nucleic acid elution method according to claim 5, wherein the hydrophobic solution contained in the nucleic acid elution liquid has a kinetic viscosity of 10 $mm^2$/s or less at 25°C.

8.   The nucleic acid elution method according to claim 5, wherein the hydrophobic solution contained in the nucleic acid elution liquid contains at least one component selected from a group consisting of silicone oil, fluorine-based oil, and liquid paraffin.

9.   The nucleic acid elution method according to claim 5, wherein, after the step of causing the nucleic acid capturing carrier and the nucleic acid elution liquid to contact each other, the nucleic acid capturing carrier is separated from the nucleic acid elution liquid, and the nucleic acid is recovered in the polar solvent contained in the nucleic acid elution liquid.

10.   The nucleic acid elution method according to claim 5, further comprising a step of causing a biological sample prepared from an organism to be examined and the nucleic acid capturing carrier to contact each other and capturing nucleic acid derived from the biological sample on the nucleic acid capturing carrier.

11.   A nucleic acid treatment apparatus comprising:

   a reaction vessel adapted to cause a nucleic acid capturing carrier capturing nucleic acid and a nucleic acid elution liquid to contact each other; and
   a separation device adapted to separate any one of the nucleic acid capturing carrier and the nucleic acid elution liquid from the reaction vessel,

wherein the nucleic acid elution liquid contains:
a polar solvent capable of dissolving the nucleic acid; and
a hydrophobic solution capable of performing liquid-liquid phase separation between the hydrophobic solution and the polar solvent.

**12.** The nucleic acid treatment apparatus according to claim 11, wherein:

the nucleic acid capturing carrier is a magnetic bead,
the separation device includes a magnetic body part containing a magnetic material, and
the separation device attracts the nucleic acid capturing carrier to the magnetic body part and separates the nucleic acid capturing carrier from the nucleic acid elution liquid in the vessel.

[FIG. 1]

```
┌─────────────────────────────────────┐
│                                      │        STEP 1
│     NUCLEIC ACID EXTRACTION STEP     │
│                                      │
└─────────────────────────────────────┘
                   ⇩
┌─────────────────────────────────────┐
│       STEP FOR BINDING TO NUCLEIC    │        STEP 2
│         ACID CAPTURING CARRIER       │
│                                      │
└─────────────────────────────────────┘
                   ⇩
┌─────────────────────────────────────┐
│                                      │        STEP 3
│            WASHING STEP              │
│                                      │
└─────────────────────────────────────┘
                   ⇩
┌─────────────────────────────────────┐
│      STEP FOR ELUTION FROM NUCLEIC   │        STEP 4
│         ACID CAPTURING CARRIER       │
│                                      │
└─────────────────────────────────────┘
                   ⇩
             ┌───────────┐
             │    END    │
             └───────────┘
```

[FIG. 2]

[FIG. 3]

[FIG. 4]

[FIG. 5]

[FIG. 6]

[FIG. 7]

[FIG. 8]

## INTERNATIONAL SEARCH REPORT

| International application No. |
|---|
| **PCT/JP2022/023851** |

**A.    CLASSIFICATION OF SUBJECT MATTER**

*C12Q 1/6834*(2018.01)i; *C12M 1/00*(2006.01)i
FI:    C12Q1/6834 Z; C12M1/00 A

According to International Patent Classification (IPC) or to both national classification and IPC

**B.    FIELDS SEARCHED**

Minimum documentation searched (classification system followed by classification symbols)

C12Q1/6834; C12M1/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN), JSTPlus/JMEDPlus/JST7580 (JDreamIII), Japio-GPG/FX, PubMed

**C.    DOCUMENTS CONSIDERED TO BE RELEVANT**

| Category* | Citation of document, with indication, where appropriate, of the relevant passages | Relevant to claim No. |
|---|---|---|
| X | JP 2011-516034 A (NORTHWESTERN UNIVERSITY) 26 May 2011 (2011-05-26) claims 1, 8, 9, example 4, paragraph [0084] | 1-12 |
| A | WO 2011/074456 A1 (HITACHI HIGH-TECHNOLOGIES CORPORATION) 23 June 2011 (2011-06-23) entire text, all drawings | 1-12 |
| A | JP 2015-104363 A (SEIKO EPSON CORP) 08 June 2015 (2015-06-08) entire text, all drawings | 1-12 |
| A | JP 2014-176302 A (SEIKO EPSON CORP) 25 September 2014 (2014-09-25) entire text, all drawings | 1-12 |
| A | US 2012/0077285 A1 (QIAGEN GMBH) 29 March 2012 (2012-03-29) entire text, all drawings | 1-12 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **24 August 2022** | **06 September 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

| Patent document cited in search report | | | Publication date (day/month/year) | Patent family member(s) | | | Publication date (day/month/year) |
|---|---|---|---|---|---|---|---|
| JP | 2011-516034 | A | 26 May 2011 | WO | 2009/111316 | A2 | |
| | | | | claims 1, 8, 9, example 4, p. 25, 4th paragraph | | | |
| | | | | US | 2009/0246782 | A1 | |
| | | | | US | 2011/0269190 | A1 | |
| | | | | US | 2012/0129156 | A1 | |
| | | | | US | 2011/0306109 | A1 | |
| | | | | US | 2012/0208987 | A1 | |
| | | | | US | 2013/0034845 | A1 | |
| | | | | EP | 2257802 | A1 | |
| WO | 2011/074456 | A1 | 23 June 2011 | US | 2012/0309104 | A1 | |
| | | | | entire text, all drawings | | | |
| | | | | EP | 2515121 | A1 | |
| JP | 2015-104363 | A | 08 June 2015 | US | 2015/0152479 | A1 | |
| | | | | entire text, all drawings | | | |
| JP | 2014-176302 | A | 25 September 2014 | US | 2014/0273101 | A1 | |
| | | | | entire text, all drawings | | | |
| | | | | EP | 2777811 | A1 | |
| US | 2012/0077285 | A1 | 29 March 2012 | WO | 2010/108971 | A1 | |
| | | | | EP | 2411811 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017184626 A
- JP 2016067274 A
- JP 2014093988 A
- JP 2017176023 A

**Non-patent literature cited in the description**

- *Precision Oncology*, 2020, vol. 4 (3)